# EUROPEAN PATENT SPECIFICATION

(11) **EP 2 648 808 B1**
(45) Date of publication and mention of the grant of the patent: **18.03.2015**
(21) Application number: 11807639.7
(22) Date of filing: 08.12.2011
(51) Int. Cl.: A61Q 5/02, A61Q 5/12, A61K 8/02, A61K 8/34, A61K 8/37, A61K 8/41

(54) **COMPOSITIONS COMPRISING QUATERNARY AMMONIUM COMPOUNDS**
ZUSAMMENSETZUNGEN MIT QUARTÄREN AMMONIUMVERBINDUNGEN
COMPOSITIONS COMPRENANT DES COMPOSÉS D'AMMONIUM QUATERNAIRE

(30) Priority: 10.12.2010 EP 10015509
(43) Date of publication of application: 16.10.2013
(73) Proprietor: Thor Especialidades S.A., 08297 Castellgali Barcelona (ES)
(72) Inventor: ARDERIU GIRAME, Marc, E-17538 Alp Girona (ES); CASAFONT MONCUNILL, Noemi, E-25280 Solsona Lleida (ES)
(74) Representative: Apenberg, Stefan
(86) International application number: PCT/EP2011/006186
(87) International publication number: WO 2012/076177

(56) References cited:
- EP-A1- 0 367 939
- WO-A1-00/28950
- WO-A1-03/060046
- WO-A1-2006/027214
- JP-A- 2005 206 573
- US-A- 4 886 660
- US-A- 4 891 214
- US-A1- 2003 026 774

## Description

The invention relates to the fields of chemistry and personal care products and provides additives which are useful and convenient for formulating shampoos, conditioners.

Fatty quaternary ammonium compositions (hereinafter "quats") are useful additives to hair care products such as shampoos, conditioners. U.S. Patent No. 4,891,214 issuing to Stevens et al. on January 2nd, 1999 provides an informative background into quats and their uses.

In the hair care industry, it is desirable to provide quats in compound and formulations having a high cationic activity (i. e. relative high concentration of quats to a water/solvent mixture). It is also desirable to produce compounds and formulations that, in addition to having a high cationic activity, provide for ease in commercial handling and storage.

Achieving the desired high cationic activity, while maintaining the product in the liquid state, is generally not feasible. The shipping and use of such dilute solutions of quats are undesirable. For the manufacturer of quat compositions, it is highly advantageous to prepare the quat compounds as compounded materials or formulations in form of flakes, pellets as well as having high active ingredient content, having a low setting point and good dispersibility in aqueous media.

In accordance with the prior art, the above mentioned requirements can be achieved by adding short-chain alcohols, in particular isopropanol or ethanol, in amounts of from 15 to 20% by weight. However, the short-chain alcohols are volatile organic solvents which raise significant environmental and safety concerns, not only for the manufacturer but also both for the consumer and for the company formulating the personal care products. Therefore, it is desirable to find other, non-volatile solvent/formulation systems.

Compositions containing quats and fatty alcohols, as opposed to the volatile organic solvents, do not share the same problem with volatility and can be flakeable. In fact, fatty alcohols such as cetearyl alcohol have been used as quat solvents. However, these compositions have a quat content of less than 35%. Attempts to increase the cationic activity, namely the amount of quat used, up to 35% leads to products with melting points typically over 100°C and in order to effectively handle and, ultimately, flake the material, it must often be heated to a point near, or above this temperature, in which the quat can begin to degrade. Moreover, this relatively high content of fatty alcohol (more than 65%) tends to dramatically increase the viscosity of the finished product.

As described in WO 00/28950 A1, the short-chain alcohols can be replaced by linear fatty alcohols, e. g. cetyl alcohol, lauryl alcohol, behenyl alcohol, or stearyl alcohol and alkyl glycols, such as for example, propylene glycol or 1,3-butandiol, which are additionally added in order to lower the setting point or melting point of the mixtures to temperatures below 100 °C.

The alkyl glycols mentioned in WO 00/28950 A1 could be also used alone as solvent systems for quats. However, they have a number of drawbacks. First, quats containing glycols as a solvent generally result in formulations which are not flakable. Instead, they form a so what viscous, waxy "gummy" solid. While this material may have a lower overall melting point than the fatty alcohol based solvent quats, the overall handling problems involved from this material are significantly greater.

Besides, glycols are typically coming from synthetic sources. Today's personal care market is challenged to find innovative, natural alternatives to synthetic and petroleum-based chemicals that have similar or better performance.

EP 1 269 992 B1 discloses compositions comprising quaternary ammonium compounds and at least one polyhydric alcohol having 5 to 12 carbon atoms. According to the teaching of this document, these compositions shall have low setting and melting points, good dispersability in aqueous media and have a low flashpoint. According to a preferred embodiment these compositions are free from fatty alcohols and linear or branched monoalcohols having 8 to 36 carbon atoms. The compositions disclosed in EP 1 269 992 B1, shall have low setting points and being nevertheless sufficiently hard and brittle at room temperature to allow pelleting or flaking, what solves the first drawback of the use of glycols as a sole solvent system. However, these polyhydric alcohols are also typically coming from synthetic sources.

In summary, each attempt to increase the quat content in the compositions while obtaining the desirable commercial handling properties of quats falls short in one aspect or another. Use of water as a solvent generally results in compositions with a comparatively low quat-content. Use of short-alkyl alcohols as a solvent is problematical due to their high organic volatility. Glycols, whether used alone or in combination with fatty alcohols, are generally petrochemical derivatives and unpopular in personal care applications. The use of solvent systems comprising long chain alkyl fatty alcohols, what leads to products with better environmental profile, results in low cationic activity of the quats and requires heating the resulting product to a point in which the quats begin to degrade.

Until now, there exists no quat compound or quat composition which exhibits all of the favourable properties described above, moreover, until now there existed no quat composition comprising at least one glycerol monoester of a C₁₂₋₂₄ fatty acid and fatty alcohols, all coming from renewable resources, as a solvent system.

Surprisingly it has now been found that compositions comprising quaternary ammonium compounds, at least one glycerol monoester of a C₁₂₋₂₄ fatty acid and fatty alcohols generally result in formulations which have low setting and melting points, good dispersability in aqueous media by maintaining the cationic activity of the quat, wherein the amount of the quat in the composition is particularly preferably higher than 40% and more preferably at least 50% by weight. Surprisingly, the concentrated compositions of the present invention can be free from short chain alcohols, in particular isopropanol and glycols such as for example propylene glycol or 1,3-butandiol. According to a preferred embodiment, the novel compositions are thus formulated with only solvents based from renewable resources, achieve a high active matter of about 50% by weight and are thus highly suitable for formulating quaternary ammonium compounds for personal care applications.

Hence, the invention provides compositions comprising, based on the finished composition,
a) 40 to 60% by weight of at least one quaternary compound according to formula (1) 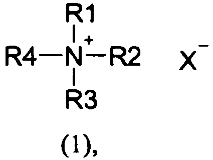 wherein R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a number from 1 to 8, and
   R₂, R₃ and R₄ are independently of one another, may be identical or and are a -CH₃, CH₃CH₂-, CH₃CH₂CH₂-, CH₃CH₂CH₂CH₂-, -CH₂CH₂OH, or-CH₂CH(OH)CH₂OH group, and
   X- is an anion selected from the group consisting of chloride, iodide, bromide, methosulfate, hydrogensulfate, lactate, citrate, carbonate and bicarbonate or mixtures thereof, and
b) 15 to 25% by weight of at least one glycerol monoester selected from the group consisting of glycerol monoester of a C₁₂₋₂₄ fatty acid, and
c) 25 to 35% by weight of a mixture of fatty alcohols having 10 to 20 carbon atoms, characterised in that said mixture of fatty alcohols comprises:
   (ca) at least one fatty alcohol having 10 to 14 carbon atoms, and
   (cb) at least one fatty alcohol having 16 to 20 carbon atoms,
   wherein the weight ratio of (ca) to (cb) ranges from 1,0 to 1,5.

The relative portions of a) to b) to c) depends somewhat on the quat used and the glycerol monoester and fatty alcohol mixture used. The fluidity of the composition of the invention is important from a manufacturing standpoint because without the proper final commercial handling properties, it will be extremely difficult to produce a commercially feasible product. During manufacture, the product has to be stirred, heated, cooled as needed and often transferred in a fluid state to a flaking or a pastillation line. All of this needs to be achieved at commercially viable temperatures, temperatures which will not degrade the quat.

The proportion of quaternary ammonium compounds (a) based on the finished composition is 40 to 60 % per weight, particularly preferably 45 to 55 % by weight, especially preferably 50 % by weight. Surprisingly it has been found, that the compositions can advantageously have high portions by weight of quaternary ammonium compounds (a) coupled with simultaneously low melting and setting points.

Quaternary ammonium compounds a) are preferably (C₁₂-C₃₆)-, preferably (C₁₄-C₂₄)-, practically preferably (C₁₆-C₂₄)-alkyltrimethylamonium compounds. Particular preference is given to alkyltrimethylammonium compounds in which the alkyl radical is a behenyl, erucyl, cetyl or stearyl radical.

The anion X- in Formula (1) maybe any desired charge-balancing anion; according to the invention, the anion is an anion selected from the group consisting of chloride, iodide, bromide, methosulfate, hydrogensulfate, lactate, citrate, carbonate and bicarbonate or a mixture thereof.

A very particularly suitable quaternary ammonium compound a) is behenyltrimethylammonium chloride.

The proportion of glycerol monoesters or the proportion of the glycerol monoester b) is, based on the finished compositions, 15 to 25 % by weight, particularly preferably 17 to 23 % by weight, especially preferably 20% by weight.

Glycerol monoesters selected from the group consisting of glycerol monoester of one or more C₁₂₋₂₄ fatty acid(s), b), are to be understood as meaning fatty acid monoesters with glycerol, wherein the fatty acid is a saturated or unsaturated fatty acid. Preferably the fatty acid is selected from the group consisting of lauric acid, myristic acid, palmitic acid, stearic acid, arachidic acid, myristoleic acid, palmitoleic acid, sapienic acid, oleic acid and arichidonic acid. Hence, according to a preferred embodiment of the invention, glycerol monoesters can include glycerol monostearate, glycerol monopalmitate, glycerol arachidate, glycerol oleate, glycerol laurate and mixtures thereof. According to a preferred embodiment of the invention, the glycerol monoester, constituent b) of the composition of the invention, is selected from the group consisting of glycerol monostearate and/or glycerol monopalmitate. According to a more preferred embodiment constituent b) is glycerol monostearate wherein the fatty acid is a mixture 50:50 % by weight of palmitic acid and stearic acid.

The proportion of fatty alcohols having 10 to 20 carbon atoms, constituent c) of the composition of the invention is, based on the finished compositions, 25 to 35 % by weight, particularly preferably 27 to 33 % by weight, especially preferably 30 % by weight. Constituent c) is characterized in that it comprises:
(ca) at least one fatty alcohol having 10 to 14 carbon atoms, and
(cb) at least one fatty alcohol having 16 to 20 carbon atoms,
   wherein the weight ratio of (ca) to (cb) ranges from 1,0 to 1,5, preferably from 1,0 to 1,2.

The specific ratio of constituent (ca) to constituent (cb) from 1,0 to 1,5, preferably from 1,0 to 1,2, leads to compositions with low setting and melting points, which are fluid enough to be manufactured feasible and flakeable.

According to the present invention, fatty alcohols having 10 to 20 carbon atoms, c), are mixtures selected of a the group consisting of capric alcohol (1-decanol), lauryl alcohol (1-dodecanol), myristyl alcohol (1-tetradecanol), cetyl alcohol (1-hexadecanol), stearyl alcohol (1-octadecanol), oleyl alcohol (cis-9-Octadecen-1-ol) and arachidyl alcohol (1-eicosanol). Mixtures such as cetearylalcohol, which are mixtures of cetyl- and stearyl alcohols, are also useful. According to a preferred embodiment of the invention, the fatty alcohols, compounds (c) are selected from the group consisting of 1-dodecanol, 1-tetradecanol, 1-hexadecanol and 1-octadecanol.

According to a further preferred embodiment of the invention, the composition consists of 50% by weight of behenyltrimethylammonium chloride, 20% by weight of glyceryl monostearate, 4,5% by weight of 1-hexadecanol, 10,5% by weight of 1-octadecanol, 10,5% by weight of 1-dodecanol, and 4,5% by weight of 1-tetradecanol.

The compositions of the invention preferably have setting points below 100 °C, very particularly preferably below 95 °C.

The compositions according to the invention may for example be in a form selected from the group consisting of pellets, flakes, extrudates, pastes, compacts, emulsions, dispersions, and mixtures thereof. According to a preferred embodiment the compositions are in the form of pellets or flakes. Flakes or pellets are desired in the commercial industry because they are easily handled and incorporated into desired products.

The claimed compositions are free-flowing liquids over their setting points, making them easy to manufacture and easy to transfer and pump through transfer lines to flaking or pastillation equipment when it is chilled below its melting point and either broken into easy to handle flakes or dispensed into pellets/pastilles and cooled.

Thus, the claimed compositions generally require the setting point to be below 100°C in order to facilitate the transfer to the flaking lines while maintaining the integrity of the quats in the composition. More preferably the setting point of the compositions is below 95 °C. Whether a formulation is flakable is measured by pouring a relatively thin film of the heated composition onto a metal sheet and allowing it to cool. The cooled film is then "crumbled" or "scraped" into small flakes by any type of mechanical process.

Last, a successful composition must possess two properties. First, the composition must possess the property of being easily poured onto the sheet plus forming a thin film. Second, once the composition is allowed to cool, it must brake into flakes after crumbling or scrapping. These flakes are consequently easily stored and re-melted as necessary.

Pastillation is a process in which small amounts of the desired formulation are dispensed into pastilles/pellets. These pastilles are then allowed to cool, forming a product which is in solid form but easy returned to liquid state. Whether a formulation is escapable of pastillation is measurable by distributing small amounts of the heated formulation into pastilles. These pastilles are then allowed to cool. The pastilles must be easily melted without tremendous amounts of heat, preferably below the boiling point of water.

The compositions of the present invention are generally suitable for the preparation of products / compositions comprising quaternary ammonium compounds.

The compositions are particularly suitable for the preparation of cosmetics, dermatological and pharmaceutical products. The invention accordingly also provides for the use of the compositions according to the invention for the preparation of products, preferably cosmetics, dermatological and pharmaceutical compositions in particular hair treatment compositions, comprising quaternary ammonium compounds.

Examples of preferred products are shampoos, rinse-off hair conditioners, cream rinsers, clear rinsers, hair cures, hair colorants and hair tints, permanent waiving compositions, hair gels, hair conditioners in aerosol, spray and fluid form, two-in-one shower preparations, cream shower preparations, skin care compositions, day creams, night creams, care creams, nutrient creams, body lotions and ointments.

The cosmetic, dermatological and pharmaceutical products or compositions comprise the compositions according to the invention, based on the finished product preferably in amounts of from 0.1 to 15% by weight, particularly preferably 1 to 10% by weigh.

The cosmetic, dermatological and pharmaceutical products can comprise as further auxiliaries and additives, all customary surfactants, oily substances, emulsifiers and co-emulsifiers, cationic polymers, film formers, super fatting agents, moisture donating agents, stabilizers, biogenic active ingredients, preservatives, pearlizing agents, dyes and fragrances, solvents, glycerol, hydrotrophic agents, opacifiers, thickeners, dispersions, protein derivatives, such as gelatines, collagen hydrolysates, natural and synthetic-based polypeptides, egg yolk, lecithin, lanolin and lanolin derivatives, silicones, deodorizing agents, substances with a keratolytic and keratoplastic action, enzymes, carrier substances, and antioxidants, UV-Light protection filters, pigments and metal oxides and antimicrobially effective agents.

Examples below serve to illustrate the invention but to no limit it.

### Example 1:

### Preparation:

A composition of 50% by weight of behenyltrimethylammonium chloride is prepared in a known manner by alkylation of a tertiary amine with methyl chloride in the presence of the following solvent types and ratios:

| | |
|---|---|
| Glyceryl stearate: | 20% by weight |
| C₁₈ alcohol: | 10,5% by weight |
| C₁₆ alcohol: | 4,5% by weight |
| C₁₄ alcohol: | 4,5% by weight |
| C₁₂ alcohol: | 10,5% by weight |

### Application:

### Basic Formula:

| **Phase** | **Component** | **Percentage (%wt)** |
|---|---|---|
| A | Demineralized water | Qsp 100% |
| | Tetrasodium EDTA | 0.05 |
| | Citric acid | 0.05 |
| B | Hydroxyethylcellulose (HEC) | 0.75 |
| | Glycerin | 2 |
| C | Steareth-21 | 1 |
| | Quat | Qsp 0.5% active matter |
| D | Cetyl alcohol | 2.5 |
| F | Preservative (Microcare^{®} IT, Thor) | 0.075 |
| | Fragrance | 0.5 |
| **Total** | | **100%** |

### Preparation of the composition:

a) Dissolve EDTA and Citric acid in water under stirring and begin to heat to 75°C
b) Disperse the HEC in the glycerin and add to phase A with rapid stirring
c) When the temperature reaches 75°C add phase C
d) Heat phase D to 75°C and add it to Phase A+B+C under fast stirring
e) Allow to cool down to 30°C and add phase F.

### Determination of wet combing:

### Hair preparation :

- Wet tresses in hot water (35 to 40 °C)
- Apply 1mL of Sodium laureth sulfate per tress
- Wash hair tresses, rolling 20 times in the hands
- Rinse with warm water for 1'30
- Switch between two fingers (3 times) to remove excess water

### Hair treatment:

- Apply 1mL of the product on one side of the hair tress, then spread the product from top to bottom 10 times
- Apply 1mL of product on the other side, then spread the product from top to bottom 10 times
- Rinse with warm water for 30 sec
- Switch between two fingers (3 times) to remove excess water

### Device parameters (Dia-stron MTT175):

- Combing Speed: 2000 mm/min
- Start Position: 30 mm
- Tress Length 150 mm
- Statistical analysis done using UvWin software and Microsoft Office Excel
Results: Wet combing score (maximum value gmf). Untreated hair: 250 gmf

### Determination of the viscosity:

- Viscosimeter: Brookfield LVDVI+
- Results: Viscosity (20°C after 1 week, RvM4V10 1')

### Results:

| Product | Active* (%) | Setting point (°C) | Wet combing (gmf) | Viscosity (cp) |
|---|---|---|---|---|
| Example 1 | 50 | 94.9 | 51.1 | 12060 |
| Microcare^{®} Quat BHQ** | 80 | 94.2 | 50.7 | 12800 |

| | | | | |
|---|---|---|---|---|
| * Active: Behentrimonium chloride [% by weight] ** Behentrimonium chloride pellets with a content of about 18% isopropanol from Thor Especialidades S.A. | | | | |

The product composition of the Example 1 gives similar results when comparing both wet combing and formula viscosity than a standard Behentrimonium chloride with Isopropanol.

### Comparative example 2 to 9:

| Example | Active* (%) | Glyceryl Stearate | C₁₈ alcohol [% by wt] | C₁₆ alcohol [% by wt] | C₁₄ alcohol [% by wt] | C₁₂ alcohol [% by wt] | Comments |
|---|---|---|---|---|---|---|---|
| 2 | 50 | 50 | 0 | 0 | 0 | 0 | NPF |
| 3 | 50 | 0 | 35 | 15 | 0 | 0 | NPF |
| 4 | 50 | 0 | 0 | 0 | 15 | 35 | v: 5570 cp |
| 5 | 50 | 0 | 21 | 9 | 6 | 14 | NPF |
| 6 | 50 | 20 | 21 | 9 | 0 | 0 | NPF |
| 7 | 50 | 20 | 0 | 0 | 9 | 21 | v: 8320 cp |
| 8 | 50 | 30 | 7 | 3 | 3 | 7 | NPF |
| 9 | 34 | 0 | 33 | 33 | 0 | 0 | v: 17580 |

| | | | | | | | |
|---|---|---|---|---|---|---|---|
| * Active: Behentrimonium chloride [% by weight] NPF: not production feasible (setting points higher than 100°C) v: Viscosity of the final formula (20°C after 1 week, RvM4V10 1') | | | | | | | |

The above table exemplifies several aspects of the present invention. While some example compositions are not feasible at production scale, others have different viscosities than the standard.

## Claims

1. A composition comprising, based on the finished composition,
a) 40 to 60% by weight of at least one quaternary compound according to formula (1) 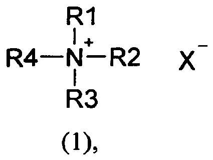 wherein R₁ is an unbranched or branched alkyl or alkenyl group having 12 to 36 carbon atoms, a group R₅CONH(CH₂)ₙ- or a group R₅COO(CH₂)ₙ-, where R₅ is an alkyl or alkenyl group having 12 to 36 carbon atoms and n is a number from 1 to 8, and
R₂, R₃ and R₄ are independently of one another, may be identical and are a -CH₃, CH₃CH₂-, CH₃CH₂CH₂-, CH₃CH₂CH₂CH₂-, -CH₂CH₂OH, or-CH₂CH(OH)CH₂OH group, and
X⁻ is an anion selected from the group consisting of chloride, iodide, bromide, methosulfate, hydrogensulfate, lactate, citrate, carbonate and bicarbonate or mixtures thereof, and
b) 15 to 25% by weight of at least one glycerol monoester selected from the group consisting of glycerol monoester of a C₁₂₋₂₄ fatty acid, and
c) 25 to 35% by weight of a mixture of fatty alcohols having 10 to 20 carbon atoms,
**characterised in that** said mixture of fatty alcohols comprises:
(ca) at least one fatty alcohol having 10 to 14 carbon atoms, and
(cb) at least one fatty alcohol having 16 to 20 carbon atoms,
wherein the weight ratio of (ca) to (cb) ranges from 1,0 to 1,5.

2. The composition as claimed in claim 1, wherein the quaternary ammonium compounds, compounds a), are (C₁₂-C₃₆)-, preferably (C₁₄-C₂₄)-, practically preferably (C₁₆-C₂₄)-alkyltrimethylammonium compounds.

3. The composition as claimed in claim 1 or 2, wherein the quaternary ammonium compound, compound a), is behenyltrimethylammonium chloride.

4. The composition as claimed in claims 1 to 3, wherein the at least one glycerol monoester, compound b), is glycerol mono stearate.

5. The composition as claimed in claims 1 to 4, wherein the fatty alcohols, compounds c), are selected from the group consisting of 1-dodecanol, 1-tetradecanol, 1-hexadecanol and 1-octadecanol.

6. The composition as claimed in claims 1 to 5, wherein the composition consists of 50% by weight of behenyltrimethylammonium chloride, 20% by weight of glyceryl monostearate, 4,5% by weight of 1-hexadecanol, 10,5% by weight of 1-octadecanol, 10,5% by weight of 1-dodecanol, and 4,5% by weight of 1-tetradecanol.

7. The composition as claimed in claims 1 to 6, which is in a form of pellets or flakes.

8. The composition as claimed in claims 1 to 7, having a setting point of less than 95°C.

## Patentansprüche

1. Eine Zusammensetzung umfassend, bezogen auf die Gesamtzusammensetzung,
a) 40 bis 60 Gew.-% von zumindest einer quaternären Verbindung gemäß der Formel (1) 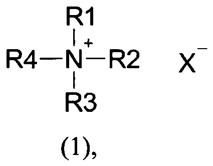 wobei R₁ eine unverzweigte oder verzweigte Alkyl- oder Alkenylgruppe mit 12 bei 36 Kohlenstoffatomen, eine Gruppe R₅CONH(CH₂)ₙ- oder eine Gruppe R₅COO(CH₂)ₙ- ist, wobei R₅ eine Alkyl oder Alkenylgruppe mit 12 bis 36 Kohlenstoffatomen ist und n eine Zahl von 1 bis 8 ist, und
R₂, R₃ und R₄ unabhängig voneinander gleich sein können und eine -CH₃, CH₃CH₂-, CH₃CH₂CH₂-, CH₃CH₂CH₂CH₂-, -CH₂CH₂OH, oder - CH₂CH(OH)CH₂OH Gruppe sind, und
X- ein Anion ist, ausgewählt aus der Gruppe bestehend aus Chlorid, Iodid, Bromid, Methosulfat, Hydrogensulfat, Lactat, Citrat, Carbonat, und Bicarbonat oder Mischungen davon, und
b) 15 bis 25 Gew.-% von zumindest einem Glycerinmonoester ausgewählt aus der Gruppe bestehend aus Glycerinmonoestern einer C₁₂₋₂₄ Fettsäure, und
c) 25 bis 35 Gew.-% einer Mischung von Fettalkoholen mit 10 bis 20 Kohlenstoffatomen,
**dadurch gekennzeichnet, dass** die Mischung von Fettalkohlen enthält:
(ca) zumindest einen Fettalkohl mit 10 bis 14 Kohlenstoffatomen und
(cb) zumindest einen Fettalkohol mit 16 bis 20 Kohlenstoffatomen, wobei das Gewichtsverhältnis von (ca) zu (cb) im Bereich von 1,0 bis 1,5 liegt.

2. Zusammensetzung gemäß Anspruch 1, wobei die quaternären Ammoniumverbindungen, Verbindungen (a), (C₁₂-C₃₆)-, bevorzugt (C₁₄-C₂₄)-, besonders bevorzugt (C₁₆-C₂₄)-Alkyltrimethylammoniumverbindungen sind.

3. Zusammensetzung gemäß Anspruch 1 oder 2, wobei die quaternäre Ammoniumverbindung, Verbindung (a), Behenyltrimethylammoniumchlorid ist.

4. Zusammensetzung gemäß einem der Ansprüche 1 bis 3, wobei der zumindest eine Glycerinmonester, Verbindung (b), Glycerinmonostearat ist.

5. Zusammensetzung gemäß einem der Ansprüche 1 bis 4, wobei die Fettalkohole, Verbindungen (c), ausgewählt sind aus der Gruppe bestehend aus 1-Dodekanol, 1-Tetradekanol, 1-Hexadekanol und 1-Oktadekanol.

6. Zusammensetzung gemäß einem der Ansprüche 1 bis 5, wobei die Zusammensetzung aus 50 Gew.-% Behenyltrimethylammoniumchlorid, 20% Gew.-% Glycerinmonostearat, 4,5 Gew.-% 1-Hexadekanol, 10,5% Gew.-% 1-Oktadekanol, 10,5 Gew.-% 1-Dodekanol, und 4,5 Gew.-% 1-Tetradekanol besteht.

7. Zusammensetzung gemäß einem der Ansprüche 1 bis 6, die in Form von Pellets oder Flocken vorliegt.

8. Zusammensetzung gemäß einem der Ansprüche 1 bis 7, die einen Absetzpunkt bei weniger als 95 °C aufweist.

## Revendications

1. Composition comprenant, sur la base de la composition finie,
a) de 40 à 60% en poids d'au moins un composé quaternaire selon la formule (1) 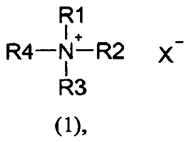 dans laquelle R₁ est un groupement alkyle ou alcényle non ramifié ou ramifié ayant de 12 à 36 atomes de carbone, un groupement R₅CONH(CH₂)ₙ- ou un groupement R₅COO(CH₂)ₙ-, où R₅ est un groupement alkyle ou alcényle ayant de 12 à 36 atomes de carbone et n est un nombre allant de 1 à 8, et
R₂, R₃ et R₄ sont indépendamment les uns des autres, peuvent être identiques et sont un groupement -CH₃, CH₃CH₂-, CH₃CH₂CH₂-, CH₃CH₂CH₂CH₂-, -CH₂CH₂OH ou -CH₂CH(OH)CH₂OH, et X⁻ est un anion choisi dans le groupe constitué par le chlorure, l'iodure, le bromure, le méthosulfate, l'hydrogénosulfate, le lactate, le citrate, le carbonate et le bicarbonate, ou des mélanges de ceux-ci, et
b) de 15 à 25% en poids d'au moins un monoester de glycérol choisi dans le groupe constitué par un monoester de glycérol d'un acide gras en C₁₂₋₂₄, et
c) de 25 à 35% en poids d'un mélange d'alcools gras ayant de 10 à 20 atomes de carbone,
**caractérisé en ce que** ledit mélange d'alcools gras comprend :
(ca) au moins un alcool gras ayant de 10 à 14 atomes de carbone, et
(cb) au moins un alcool gras ayant de 16 à 20 atomes de carbone,
où le rapport pondéral de (ca) à (cb) va de 1,0 à 1,5.

2. Composition selon la revendication 1, dans laquelle les composés d'ammonium quaternaire, les composés a), sont des composés de (C₁₂-C₃₆)-, préférablement de (C₁₄-C₂₄)-, particulièrement préférablement de (C₁₆-C₂₄)-alkyltriméthylammonium.

3. Composition selon la revendication 1 ou 2, dans laquelle le composé d'ammonium quaternaire, le composé a), est le chlorure de béhényltriméthylammonium.

4. Composition selon les revendications 1 à 3, dans laquelle le au moins un monoester de glycérol, le composé b), est le monostéarate de glycérol.

5. Composition selon les revendications 1 à 4, dans laquelle les alcools gras, les composés c), sont choisis dans le groupe constitué par le 1-dodécanol, le 1-tétradécanol, le 1-hexadécanol et le 1-octadécanol.

6. Composition selon les revendications 1 à 5, dans laquelle la composition est constituée de 50% en poids de chlorure de béhényltriméthylammonium, 20% en poids de monostéarate de glycérol, 4,5% en poids de 1-hexadécanol, 10,5% en poids de 1-octadécanol, 10,5% en poids de 1-dodécanol, et 4,5% en poids de 1-tétradécanol.

7. Composition selon les revendications 1 à 6, qui se trouve sous la forme de pastilles ou de paillettes.

8. Composition selon les revendications 1 à 7, ayant un poids de solidification inférieur à 95°C.
